# EUROPEAN PATENT APPLICATION

(11) **EP 4 850 740 A1**
(43) Date of publication of application: **09.12.2026**
(21) Application number: 24921176.4
(22) Date of filing: 16.04.2024
(51) Int. Cl.: A61M 35/00, B05B 15/00, A24F 40/00

(54) **ATOMIZATION APPARATUS**

(30) Priority: 31.01.2024 CN 202420253762 U
(71) Applicant: Shenzhen Moore Vaporization Health & Medical Technology Co., Ltd., Shenzhen, Guangdong 518102 (CN)
(72) Inventor: FENG, Ke, Shenzhen, Guangdong 518102 (CN); ZHAO, Tao, Shenzhen, Guangdong 518102 (CN); PENG, Weisong, Shenzhen, Guangdong 518102 (CN); ZHENG, Xin, Shenzhen, Guangdong 518102 (CN); HUANG, Lianhui, Shenzhen, Guangdong 518102 (CN); XIE, Faming, Shenzhen, Guangdong 518102 (CN)
(74) Representative: Winter, Brandl - Partnerschaft mbB
(86) International application number: PCT/CN2024/087902
(87) International publication number: WO 2025/161132

(57) **Abstract**

This application provides an atomization apparatus, to resolve a problem in the prior art that an atomization mechanism is fixed in a housing and is inconvenient to replace the atomization mechanism. The atomization apparatus includes the housing and the atomization mechanism. The housing has an accommodating cavity and a mounting opening. The atomization mechanism is detachably arranged in the accommodating cavity. In this way, when the atomization mechanism is replaced, the atomization mechanism may be taken out from the mounting opening, and an atomization mechanism to be replaced is mounted in the accommodating cavity through the mounting opening, so that the atomization apparatus may have a function of replacing the atomization mechanism, to resolve a problem that the atomization mechanism is inconvenient to be replaced for an existing cosmetic instrument.

## Description

### CROSS-REFERENCE TO RELATED APPLICATIONS

This application claims priority to Chinese Patent Application No. 2024202537621 filed on January 31, 2024, which is incorporated herein by reference in its entirety.

### TECHNICAL FIELD

This application relates to the field of atomization apparatus technologies, and in particular, to an atomization apparatus.

### BACKGROUND

A cosmetic instrument sprays a cosmetic liquid onto, for example, skin, so that the skin better absorbs the cosmetic liquid. The cosmetic instrument includes a housing and an atomization mechanism (configured to spray a cosmetic instrument). The atomization mechanism is fixedly mounted in the housing. When the atomization mechanism is replaced, it is inconvenient to replace the atomization mechanism.

### SUMMARY

A main technical problem to be resolved in this application is to provide an atomization apparatus, to resolve a problem in the prior art that an atomization mechanism is fixed in a housing and is inconvenient to replace the atomization mechanism.

To resolve the foregoing technical problem, a first technical solution adopted in this application is to provide an atomization apparatus. The atomization apparatus includes a housing and an atomization mechanism. The housing has an accommodating cavity and a mounting opening. The atomization mechanism is detachably arranged in the accommodating cavity. In this way, when the atomization mechanism is replaced, the atomization mechanism may be taken out from the mounting opening, and an atomization mechanism to be replaced is mounted in the accommodating cavity through the mounting opening, so that the atomization apparatus may have a function of replacing the atomization mechanism, to resolve a problem that the atomization mechanism is inconvenient to be replaced for an existing cosmetic instrument.

In addition, when the existing cosmetic instrument uses different cosmetic liquids, the old cosmetic liquid in the atomization mechanism needs to be first rinsed, and then a new cosmetic instrument needs to be injected into the atomization mechanism. However, the atomization mechanism is detachably arranged in the accommodating cavity, and different cosmetic liquids may be respectively injected into a plurality of atomization mechanisms, thereby reducing deterioration, pollution, or the like caused by microbes in the atomization mechanisms.

In some embodiments, the atomization apparatus further includes an air supply mechanism, and the air supply mechanism is mounted in the accommodating cavity. An air supply interface protrudes from a first outer wall of the atomization mechanism. The air supply mechanism is in communication with the air supply interface, and is configured to supply air to the atomization mechanism.

In some embodiments, the atomization mechanism includes an atomization assembly and an atomization cartridge in communication with each other. A liquid in the atomization cartridge can enter the atomization assembly. A second outer wall of the atomization assembly and a third outer wall of the atomization cartridge form a first outer wall, and the air supply interface protrudes from the second outer wall.

In some embodiments, the atomization assembly is detachably connected to the atomization cartridge.

In some embodiments, the atomization apparatus further includes a liquid pushing mechanism mounted in the accommodating cavity. The liquid pushing mechanism is configured to push the liquid in the atomization cartridge into the atomization assembly.

In some embodiments, the liquid pushing mechanism includes a push rod, a screw rod, a driving member, and a pressing plate. The driving member is connected to the screw rod, and the screw rod is threadedly connected to the push rod. The driving member is configured to drive the screw rod to rotate, to cause the push rod to move along an axis of the screw rod. The push rod is configured to push the liquid in the atomization cartridge into the atomization assembly. The pressing plate is arranged on the driving member, and can abut against the push rod.

In some embodiments, the atomization assembly, the atomization cartridge, and the liquid pushing mechanism are sequentially arranged along an axial direction.

In some embodiments, a piston is arranged in the atomization cartridge. The liquid pushing mechanism is configured to push the piston to move.

In some embodiments, the atomization mechanism further includes a flip cover mechanism, the flip cover mechanism covers a flip cover and a limiting mechanism, and the flip cover is rotatably connected to the housing and is configured to cover the mounting opening. The limiting mechanism has a limiting state and a release state. A linkage fit between the flip cover and the limiting mechanism is non-connected coupling when the flip cover rotates, to cause the flip cover to switch a state of the limiting mechanism.

In some embodiments, the accommodating cavity is provided with a positioning groove mated with the air supply interface.

### BRIEF DESCRIPTION OF THE DRAWINGS

To describe technical solutions of embodiments of this application more clearly, drawings required for describing the embodiments are briefly described below. Apparently, the drawings in the following description show only some embodiments of this application, and a person of ordinary skill in the art can derive other drawings from the drawings without creative efforts.
FIG. 1 is a schematic structural diagram of an atomization device according to this application;
FIG. 2 is a schematic structural diagram of a state of an atomization apparatus according to this application;
FIG. 3 is an enlarged view of Q in FIG. 2;
FIG. 4 is a schematic structural diagram of still another state of an atomization apparatus according to this application;
FIG. 5 is an enlarged view of P in FIG. 4;
FIG. 6 is a schematic structural diagram of another state of an atomization apparatus according to this application;
FIG. 7 is an enlarged view of W in FIG. 6;
FIG. 8 is a schematic structural diagram of yet another state of an atomization apparatus according to this application;
FIG. 9 is an enlarged view of R in FIG. 8;
FIG. 10 is a schematic structural diagram when an atomization mechanism in an atomization apparatus according to this application is ejected;
FIG. 11 is a schematic structural diagram of an atomization apparatus with a part of a housing removed according to this application;
FIG. 12 is a schematic structural diagram of an atomization apparatus with an entirety of a housing removed according to this application;
FIG. 13 is a schematic structural diagram of an atomization mechanism and a housing according to this application;
FIG. 14 is a schematic structural diagram of an atomization mechanism according to this application; and
FIG. 15 is a schematic structural diagram of an atomization mechanism according to this application.

In the figure: 1. Charger; 2. Atomization apparatus; 20. First elastic member; 21. Flip Cover; 211. Cover body; 212. Trigger portion; 22. Atomization mechanism; 221. Atomization assembly; 222. Atomization cartridge; 223. Groove; 224. Air supply interface; 2241. Slot; 225. Atomization cavity; 226. Piston; 227. First outer wall; 228. Second outer wall; 229. Third outer wall; 23. Housing; 231. Accommodating cavity; 232. Positioning groove; 24. Limiting mechanism; 241. Rotating assembly; 2411. Sliding member; 2412. Rotating member; 242. Elastic assembly; 2421. Third elastic member; 2422. Second elastic member; 25. Air supply mechanism; 26. Liquid pushing mechanism; 27. Circuit board; 28. Power supply; and 29. First sensor.

### DETAILED DESCRIPTION

Solutions in embodiments of this application are described in detail below with reference to drawings in this specification.

In the following descriptions, specific details such as a specific system structure, an interface, and a technology are provided for the purpose of description rather than limitation, to facilitate thorough understanding of this application.

Technical solutions in embodiments of this application are clearly and completely described below with reference to accompanying drawings in the embodiments of this application. Apparently, the described embodiments are merely some rather than all of the embodiments of this application. All other embodiments obtained by a person of ordinary skill in the art based on the embodiments of this application without creative efforts fall within the protection scope of this application.

Terms "first", "second", and "third" in this application are merely intended for a purpose of description, and shall not be understood as an indication or implication of relative importance or implicit indication of a quantity of indicated technical features. Therefore, features defining "first", "second", and "third" may explicitly or implicitly include at least one of the features. In description of this application, "a plurality of" means at least two, such as two and three, unless otherwise specifically defined. All directional indications (for example, up, down, left, right, front, back) in the embodiments of this application are only used for explaining relative position relationships, movement situations, or the like between the various components in a specific posture (as shown in the accompanying drawings). If the specific posture changes, the directional indications change accordingly. In addition, terms "include", "have", and any variant thereof are intended to cover a non-exclusive inclusion. For example, a process, a method, a system, a product, or a device that includes a series of steps or units is not limited to the listed steps or units, and instead, further optionally includes a step or unit that is not listed, or further optionally includes another step or unit that is intrinsic to the process, the method, the product, or the device.

The "embodiment" mentioned in the specification means that particular features, structures, or characteristics described with reference to the embodiments may be included in at least one embodiment of this application. The phrase appearing at various locations in this specification does not necessarily refer to a same embodiment, and is not an independent or alternative embodiment mutually exclusive of another embodiment. A person skilled in the art explicitly or implicitly understands that the embodiments described in this specification may be combined with other embodiments.

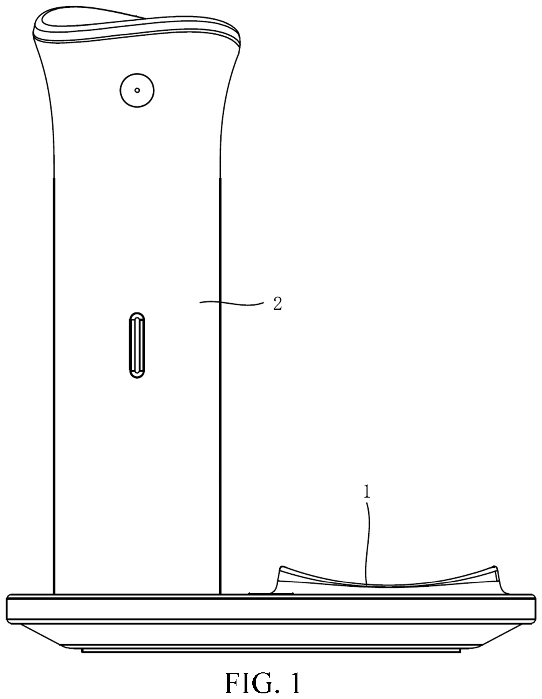

An embodiment of the present disclosure provides an atomization device. Refer to FIG. 1. The atomization device may include an atomization apparatus 2 and a charger 1. The charger 1 is configured to charge the atomization apparatus 2. In some embodiments, a charging interface is arranged on the atomization apparatus 2. An insertion end of the charger 1 is inserted into the charging interface, to charge a power supply 28 of the atomization apparatus 2.

In some other examples, the charger 1 charges the power supply 28 of the atomization apparatus 2 in a wireless manner. For example, the charger 1 charges the power supply 28 of the atomization apparatus 2 in a Bluetooth charging manner.

In some embodiments, the atomization device further includes another instrument. For example, the another instrument may be a standby charger 1, a mounting box (the mounting box is configured to assemble the atomization apparatus 2 and the charger 1 above together, which is convenient for transportation and the like), or the like.

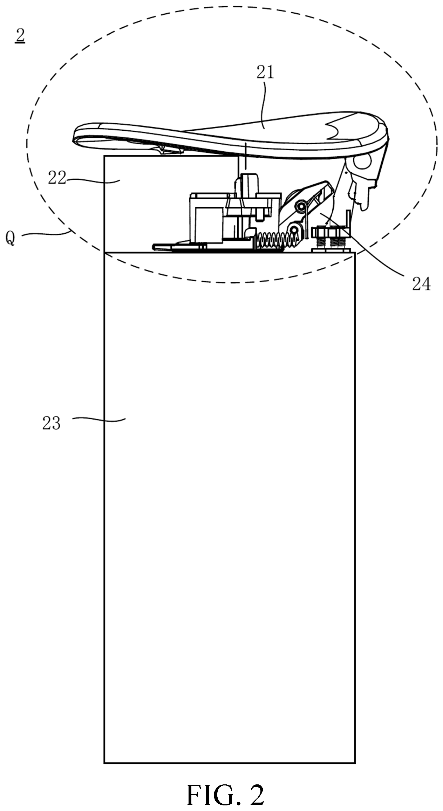

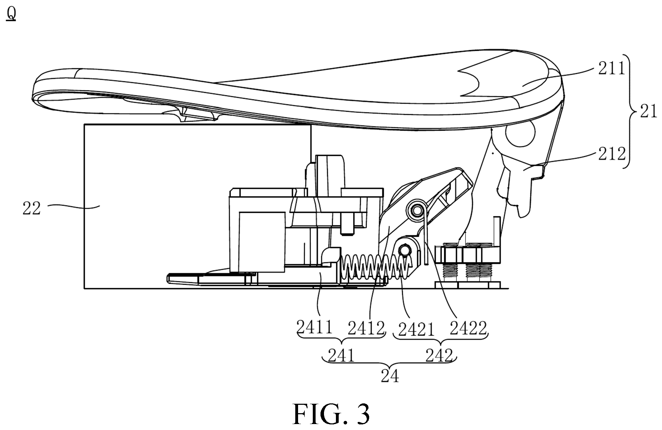

Refer to FIG. 2 and FIG. 3. The atomization apparatus may include a housing 23, an atomization mechanism 22, and a flip cover mechanism.

The housing 23 has an accommodating cavity 231 and a mounting opening. The mounting opening is in communication with the accommodating cavity. In this way, the atomization mechanism 22 may be mounted in the accommodating cavity through the mounting opening. For example, a liquid pushing assembly below, an air supply mechanism 25 below, the power supply 28, and the like may be all mounted in the accommodating cavity through the mounting opening. For another example, the housing further has a receiving cavity. The receiving cavity is also in communication with the mounting opening. The liquid pushing assembly below, the air supply mechanism 25 below, the power supply 28, and the like may be all mounted in the receiving cavity through the mounting opening. For another example, the housing further has a receiving cavity and a placement opening. The receiving cavity is also in communication with the placement opening. The liquid pushing assembly below, the air supply mechanism 25 below, the power supply 28, and the like may be all mounted in the receiving cavity through the placement opening.

The atomization mechanism 22 is configured to spray an atomization liquid (which may be referred to as an atomization substrate). For example, the liquid may be a medicinal liquid, a nutrient solution, a cosmetic liquid, an essential liquid, an essential lotion, a sterilizing liquid, an insecticide, a pesticide, or the like, so that the atomization apparatus 2 may be applied to a plurality of fields (for example, a medical field, a cosmetic field, and an agricultural field). For example, when the atomization apparatus 2 is used in the cosmetic field, the atomization apparatus 2 may be referred to as a cosmetic instrument, an atomizer, or the like. The atomization mechanism 22 may include an atomization assembly 221 and an atomization cartridge 222 that are detachably connected (for example, snap-fitted with) to each other. The atomization assembly 221 is in communication with the atomization cartridge 222. The atomization cartridge 222 is configured to store a liquid (which may also be referred to as a substrate). The liquid in the atomization cartridge 222 flows into the atomization assembly 221, and is atomized and sprayed by the atomization assembly 221.

The atomization mechanism 22 is detachably mounted in the accommodating cavity. For example, the atomization mechanism 22 may be detachably mounted in the accommodating cavity through a snap, a bolt, a latch, or the like. For another example, the limiting mechanism 24 limits the atomization mechanism 22 in the accommodating cavity. In this way, when the atomization mechanism is replaced, the atomization mechanism 22 may be taken out from the mounting opening, and an atomization mechanism 22 to be replaced is mounted in the accommodating cavity through the mounting opening, thereby resolving a problem that the atomization mechanism is inconvenient to be replaced for an existing cosmetic instrument. A plurality of atomization mechanisms 22 may be provided. In this way, different cosmetic liquids may be respectively injected into a plurality of atomization mechanisms 22, thereby reducing deterioration, pollution, or the like caused by microbes in the atomization mechanisms.

The flip cover mechanism may be applied to a type of apparatus having a housing 23 with a mounting opening, and can be fitted with a member to be mounted, to mount the member to be installed in the housing 23 or detach the member from the housing 23. For example, the apparatus may be the atomization apparatus 2. To be specific, the flip cover mechanism is used on the atomization apparatus 2. In this case, the member to be mounted may be the atomization mechanism 22. For another example, the apparatus may be a display, a main chassis, or the like. In this specification, an example in which the flip cover mechanism may be used on the atomization apparatus 2, and the member to be mounted may be the atomization mechanism 22 is used for description.

The flip cover mechanism may include a flip cover 21 and a limiting mechanism 24.

The flip cover 21 is configured to cover the mounting opening, and the mounting opening may be opened and closed by rotating the flip cover 21. A material of the flip cover 21 may be a metal material, for example, iron and copper, and may also be a non-metallic material, for example, plastic.

The flip cover 21 is rotatably connected to the housing 23. For example, the flip cover 21 is hingedly connected to the housing 23. The limiting mechanism 24 is movably arranged in the housing 23. In this way, a linkage fit is formed between the limiting mechanism 24 and the flip cover 21, so that the limiting mechanism 24 can be switched to a corresponding state by rotating the flip cover 21.

The limiting mechanism 24 has a limiting state and a release state. The limiting state may be understood as a state in which the limiting mechanism 24 is configured to limit the atomization mechanism 22 in the housing 23. To be specific, the limiting mechanism 24 is fitted with a limiting portion of the atomization mechanism 22, to limit the atomization mechanism 22 in the accommodating cavity.

The release state may be understood as a state in which the limiting mechanism 24 is configured to release the atomization mechanism 22. To be specific, the flip cover 21 (for example, in a manner of driving, pulling, or triggering) causes the limiting mechanism to release a limitation on the limiting portion. In other words, in this specification, only when the limiting mechanism 24 is fitted with the limiting portion of the atomization mechanism 22, the state of the limiting mechanism 24 is referred to as the limiting state. Any state of the limiting mechanism 24 other than the limiting state may be referred to as the release state. For example, the release state of the limiting mechanism 24 may be a state of the limiting mechanism 24 when the limiting mechanism 24 is in contact with a non-limiting portion of the atomization mechanism 22. For another example, the release state may also be a state of the limiting mechanism 24 when the limiting mechanism 24 is decoupled (which may be referred to as uncoupled or separated) from the atomization mechanism 22.

The linkage fit between the flip cover 21 and the limiting mechanism 24 is non-connected coupling when the flip cover 21 rotates, to cause the flip cover 21 to switch a state of the limiting mechanism 24. In this way, the non-connected coupling may avoid the flip cover 21 from being always coupled with the limiting mechanism 24 during rotation (for example, an opening process or a closing process of the flip cover 21), so that operation manners between the flip cover 21 and the limiting mechanism 24 can be more (for example, an opening free stroke and an opening coupling stroke below). A state of the limiting mechanism 24 is switched, so that the limiting mechanism 24 can fix the atomization mechanism 22 in the housing 23, or release a limitation on the atomization mechanism 22, thereby facilitating installation and detachment of the atomization mechanism 22. The non-connected coupling and the switching a state of the limiting mechanism 24 may omit a step in which the limiting mechanism 24 is separately operated to release or limit the atomization mechanism 22, to resolve a problem about a step in which the limiting mechanism 24 needs to be separately operated after the flip cover 21 rotates.

The linkage fit between the flip cover 21 and the limiting mechanism 24 is non-connected coupling, which may be understood as that when the flip cover 21 rotates, the linkage fit may be divided into a decoupling stroke (for example, an opening free stroke, an opening empty stroke, a closing free stroke, and a closing empty stroke below) and a coupling stroke (for example, an opening coupling stroke and a closing coupling stroke below).

The flip cover 21 switches a state of the limiting mechanism 24, which may be understood as that a state of the limiting mechanism 24 is switched from the limiting state to the release state, or a state of the limiting mechanism 24 is switched from the release state to the limiting state.

The flip cover 21 rotates, which may include the opening process of the flip cover 21 and the closing process of the flip cover 21.

In some embodiments, in the opening process of the flip cover 21, the linkage fit between the flip cover 21 and the limiting mechanism 24 is non-connected coupling, so that the flip cover 21 switches a state of the limiting mechanism 24 from the limiting state to the release state.

In this way, when the flip cover 21 is opened, the limiting mechanism 24 can release a limitation on the atomization mechanism 22 while the mounting opening is exposed, so that the atomization mechanism 22 can be conveniently taken out.

For example, in the opening process of the flip cover 21, the opening process of the flip cover 21 sequentially includes the opening coupling stroke and the opening free stroke. In this way, when the flip cover 21 is just opened, the flip cover 21 may cause the limiting mechanism 24 to release a limitation on the atomization mechanism 22.

For another example, in the opening process of the flip cover 21, the opening process of the flip cover 21 sequentially includes the opening empty stroke and the opening coupling stroke. In this way, only after the flip cover 21 is opened for a period of time or a stroke (i.e., the opening empty stroke), the flip cover 21 can cause the limiting mechanism 24 to release a limitation on the atomization mechanism 22, so that the atomization mechanism 22 can be taken out from the housing 23. When the flip cover 21 is in the opening empty stroke, the limiting mechanism 24 is in the limiting state. To be specific, the limiting mechanism 24 still limits the atomization mechanism 22, to avoid a problem that an accident is caused by a user for accidentally touching and opening the flip cover 21. In some embodiments, the opening process of the flip cover 21 further includes the opening free stroke after the opening coupling stroke. The opening free stroke is similar to the opening empty stroke. During this stroke, the flip cover 21 and the limiting mechanism 24 are in a decoupled state.

The opening empty stroke, the opening coupling stroke, and the opening free stroke of the flip cover 21 are described in detail below.

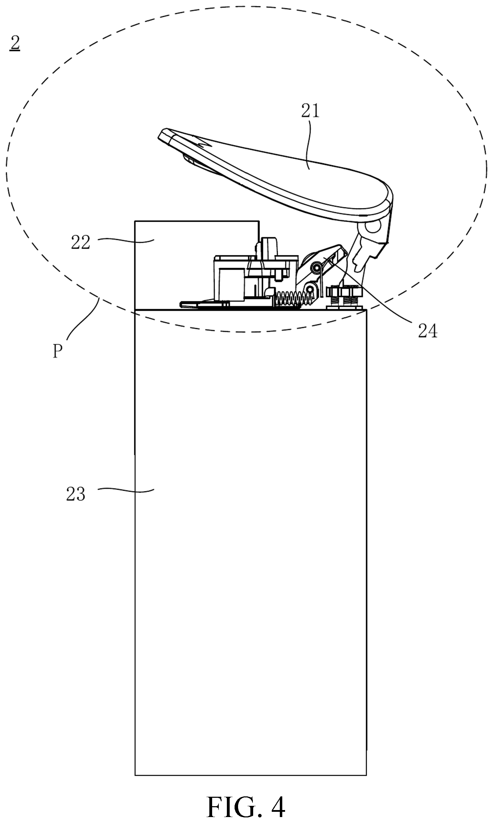

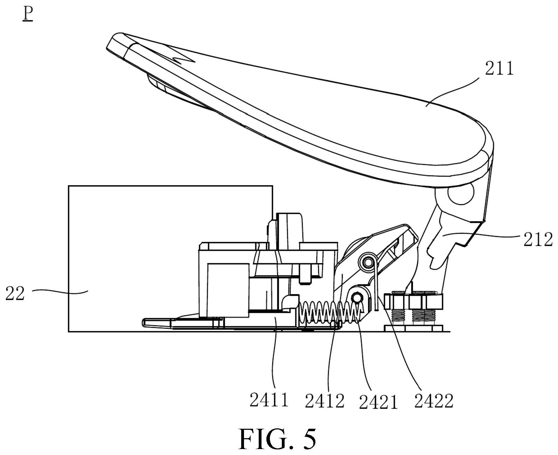

Refer to FIG. 4 and FIG. 5. During the opening empty stroke, the flip cover 21 is decoupled from the limiting mechanism 24, and the limiting mechanism 24 maintains the limiting state.

For example, the limiting mechanism 24 includes a rotating assembly 241 and an elastic assembly 242. The elastic assembly 242 is connected to the rotating assembly 241. The rotating assembly 241 is configured to be rotatably connected to the housing 23. For example, the rotating assembly 241 is rotatably connected to the housing 23. The elastic assembly 242 is configured to be connected to the housing 23. For example, the elastic assembly 242 is connected to the housing 23.

During the opening empty stroke, the flip cover 21 is decoupled from the rotating assembly 241, and the elastic assembly 242 drives the rotating assembly 241 to be in the limiting state, so that the rotating assembly 241 can continue to limit the atomization mechanism 22 in the accommodating cavity. Because the flip cover 21 is decoupled from the rotating assembly 241, to cause the rotating assembly 241 to limit the atomization mechanism 22, in this case, an elastic force of the elastic assembly 242 provides an elastic force for the rotating assembly 241. This elastic force causes the rotating assembly 22 to limit the atomization mechanism 22 in the accommodating cavity.

In some embodiments, the rotating assembly 241 only includes a rotating member 2412. The elastic assembly 242 only includes a third elastic member 2421.

In some other examples, the rotating assembly 241 includes the rotating member 2412 and a sliding member 2411. The rotating member 2412 is configured to be rotatably connected to the housing 23. For example, the rotating member 2412 is rotatably connected to the housing 23. The sliding member 2411 is configured to be slidably connected to the housing 23. For example, the sliding member 2411 is slidably connected to the housing 23 (for example, the housing 23 has a sliding groove, and the sliding member 2411 is slidably arranged in the sliding groove). The elastic assembly 242 includes a second elastic member 2422 and the third elastic member 2421. The second elastic member 2422 is connected to the rotating member 2412, and is configured to be connected to the housing 23. The third elastic member 2421 is connected to the sliding member 2411, and is configured to be connected to the housing 23. For example, both the second elastic member 2422 and the third elastic member 2421 are also connected to the housing 23. An example in which the rotating assembly 241 includes the rotating member 2412 and the sliding member 2411, and the elastic assembly 242 includes the second elastic member 2422 and the third elastic member 2421 is used for description below.

During the opening empty stroke, the flip cover 21 is decoupled from the rotating member 2412, and the third elastic member 2421 drives the sliding member 2411 to be in the limiting state. The rotating member 2412 is decoupled from the sliding member 2411, or the second elastic member 2422 drives the rotating member 2412 and the sliding member 2411 to be in a balanced state. In this way, because the rotating member 2412 is far away from the atomization mechanism 22, the sliding member 2411 is fitted with the rotating member 2412, to implement a limitation on the atomization mechanism 22 or release the limitation, so that installation of the atomization mechanism 22 and the limiting mechanism 24 has a relatively large fault tolerance rate, to better arrange the atomization mechanism 22, the limiting mechanism 24, and a component such as a battery below in the accommodating cavity. The arranged second elastic member 2422 acts on the rotating member 2412, and the third elastic member 2421 acts on the sliding member 2411, which have respective functions and do not interfere with each other.

The sliding member 2411 may be a component that can implement the foregoing functions and can slide. Therefore, a shape of the sliding member 2411 may be designed based on a requirement of the atomization apparatus 2. For example, the sliding member 2411 may be of a structure such as a sliding rod or a sliding block. The rotating member 2412 is a component that is mounted on the housing 23 and can rotate relative to the housing 23. For example, the rotating member 2412 may be of a structure such as a rod, a slender plate, or a long strip.

The sliding member 2411 is fitted with the limiting portion of the atomization mechanism 22. In some examples, the sliding member 2411 is inserted into the limiting portion of the atomization mechanism 22, to limit the atomization mechanism 22 in the accommodating cavity. In this case, the sliding member 2411 may be a latch, and the limiting portion may be a groove 223. In the limiting state, a part of the sliding member 2411 is inserted into the groove 223. In this way, a simple insertion function is used to limit the atomization mechanism 22 in the accommodating cavity. For example, an outer wall of the atomization assembly 221 has the groove 223. For another example, an outer wall of the atomization cartridge 222 has the groove 223. For another example, the groove 223 is formed between an outer bottom wall of the atomization assembly 221 and an outer top wall of the atomization cartridge 222. The second elastic member 2422 may be a torsion spring and/or a coil spring, and the third elastic member 2421 may be a spring and/or an elastic piece. In some other embodiments, the sliding member 2411 is snap-fitted with the limiting portion of the atomization mechanism 22, to limit the atomization mechanism 22 in the accommodating cavity. In this case, the limiting portion and the sliding member 2411 may be contact-type snaps. In some other embodiments, the sliding member 2411 is bonded to the limiting portion of the atomization mechanism 22, to limit the atomization mechanism 22 in the accommodating cavity. In this case, the limiting portion and the sliding member 2411 may be hook and loop fasteners or the like.

The flip cover 21 includes a cover body 211 and a trigger portion 212, and the trigger portion 212 is connected to the cover body 211. For example, the cover body 211 and the trigger portion 212 are of an integral structure. The cover body 211 may be rotatably connected to the housing 23. In this way, when the cover body 211 is opened, the trigger portion 212 rotates along with the cover body 211, to cause the trigger portion 212 to be coupled with or decoupled from the rotating member 2412. During the opening empty stroke, the flip cover 21 is decoupled from the limiting mechanism 24, which refers to that the cover body 211 and the trigger portion 212 are both decoupled from the limiting mechanism 24.

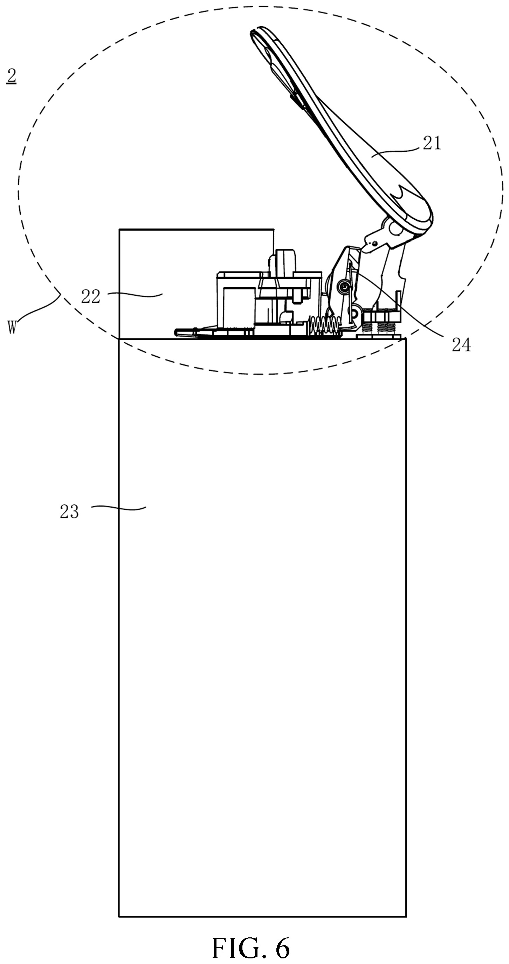

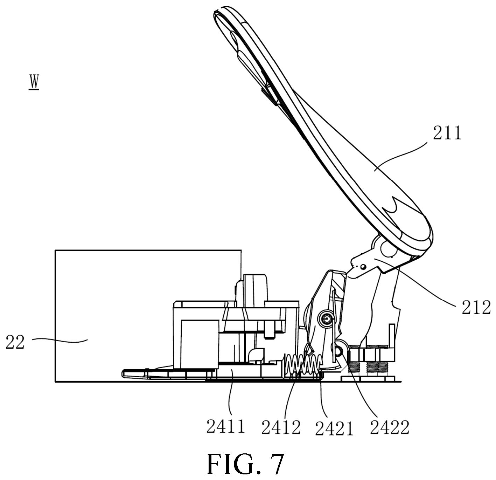

Refer to FIG. 6 and FIG. 7. During the opening coupling stroke, the flip cover 21 is coupled with the limiting mechanism 24, so that the flip cover 21 switches a state of the limiting mechanism 24 to the release state (i.e., from the limiting state to the release state).

For example, the limiting mechanism 24 includes the rotating assembly 241 and the elastic assembly 242. During the opening coupling stroke, the flip cover 21 is coupled with the rotating assembly 241, so that a state of the rotating assembly 241 is switched to the release state, and the elastic assembly 242 forces the rotating assembly 241 to restore to the limiting state. In this way, the rotating assembly 241 is fitted with the elastic assembly 242, to implement a limitation on the atomization mechanism 22. In addition, the rotating assembly 241 is fitted with the flip cover to release the limitation on the atomization mechanism 22.

From the opening empty stroke to the opening coupling stroke, a position of the rotating assembly 241 changes, to cause the elastic force of the elastic assembly 242 to increase, so that during the opening coupling stroke, the elastic force of the elastic assembly 242 can cause the rotating assembly 241 to restore to having a function of limiting a state of the atomization mechanism 22.

In some examples, the rotating assembly 241 includes the rotating member 2412 and the sliding member 2411. The elastic assembly 242 includes the second elastic member 2422 and the third elastic member 2421. During the opening coupling stroke, the flip cover 21 rotates until the flip cover 21 is coupled with the rotating member 2412, and the rotating member 2412 abuts against the sliding member 2411. The rotating member 2412 drives the sliding member 2411 to rotate to the release state. The elastic forces of the second elastic member 2422 and the third elastic member 2421 both increase, so that the increased elastic force of the second elastic member 2422 can force the sliding member 2411 to restore to having a function of limiting a state of the atomization mechanism 22. The increased elastic force of the third elastic member 2421 forces the rotating member 2412 to restore.

In some examples, the flip cover 21 includes the cover body 211 and the trigger portion 212. During the opening coupling stroke, the flip cover 21 is coupled with the limiting mechanism 24, which refers to that the trigger portion 212 is coupled with the limiting mechanism 24, and the cover body 211 is decoupled from the limiting mechanism 24. To be specific, the cover body 211 is coupled with the limiting mechanism 24 through the limiting portion.

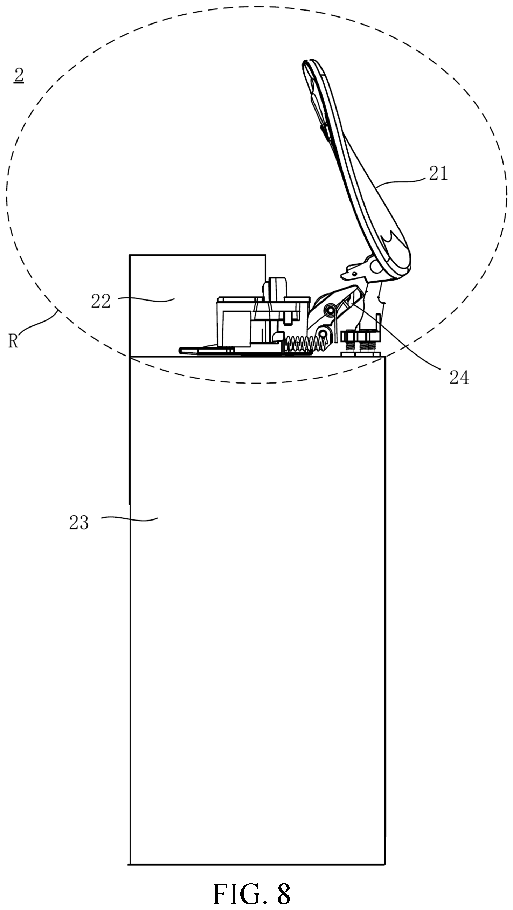

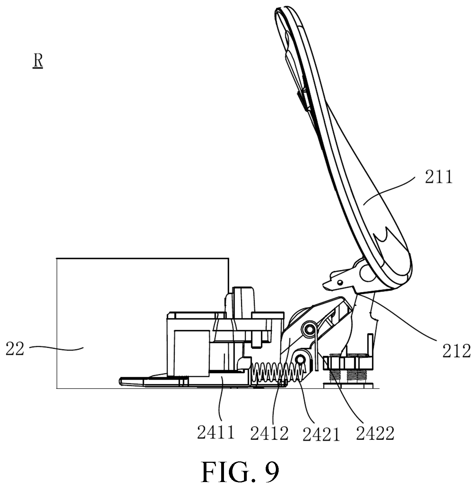

Refer to FIG. 8 and FIG. 9. During the opening free stroke, the flip cover 21 is decoupled from the limiting mechanism 24. The limiting mechanism 24 maintains the release state. In this way, the flip cover 21 is further opened, so that the mounting opening has a relatively large opening. Therefore, when the atomization mechanism 22 is taken out, the atomization mechanism 22 does not touch the flip cover 24, thereby conveniently taking out the atomization mechanism 22 from the accommodating cavity.

From the opening coupling stroke to the opening free stroke, the atomization mechanism 22 is not taken out. To be specific, the atomization mechanism is still located in the accommodating cavity. In this case, the limiting mechanism 24 is configured to abut against the non-limiting portion of the atomization mechanism 22. For example, the first elastic member causes the atomization mechanism 22 to be bounced, so that the limiting mechanism 24 abuts against the non-limiting portion of the atomization mechanism 22, thereby reducing a bouncing speed of the atomization mechanism 22.

For example, during the opening free stroke, an old atomization mechanism 22 is taken out, and a new atomization mechanism 22 is mounted. The atomization mechanism 22 is bounced by the first elastic member, to cause the limiting mechanism 24 to abut against the non-limiting portion of the atomization mechanism 22, so that during a subsequent cover closing process, the atomization mechanism 22 can be pressed while the mounting opening is covered by using a flip cover, to cause the atomization mechanism 22 to be limited by the limiting mechanism.

For another example, during the opening free stroke, the old atomization mechanism 22 is taken out, and the new atomization mechanism 22 is mounted. In this case, the atomization mechanism 22 is manually pressed, so that the limiting mechanism 24 can be directly fitted with the limiting portion of the atomization mechanism 22, to limit the atomization mechanism 22 in the accommodating cavity.

During the opening coupling stroke, the release state of the limiting mechanism 24 is that the limiting mechanism 24 is decoupled from the atomization mechanism 22. During the opening free stroke, the release state of the limiting mechanism 24 is that the limiting mechanism 24 is in contact with the non-limiting portion of the atomization mechanism 22. The non-limiting portion may be understood as an outer wall of the atomization mechanism 22, for example, an outer wall of the atomization cartridge 222, and for another example, an outer wall of the atomization assembly 221. The flip cover 21 includes the cover body 211 and the trigger portion 212. During the opening free stroke, the flip cover 21 is decoupled from the limiting mechanism 24 refers to that the trigger portion 212 and the cover body 211 are both decoupled from the limiting mechanism 24.

For example, the limiting mechanism 24 includes the rotating assembly 241 and the elastic assembly 242. During the opening free stroke, the flip cover 21 is decoupled from the rotating assembly 241, the limiting mechanism 24 maintains the release state, and the elastic assembly 242 drives the limiting mechanism 24 to abut against the non-limiting portion of the atomization mechanism 22.

In some examples, the rotating assembly 241 includes the rotating member 2412 and the sliding member 2411. The elastic assembly 242 includes the second elastic member 2422 and the third elastic member 2421. During the opening free stroke, the flip cover 21 is decoupled from the rotating member 2412, and the third elastic member 2421 drives the sliding member 2411 to abut against the non-limiting portion of the atomization mechanism 22. The rotating member 2412 is decoupled from the sliding member 2411, or the second elastic member 2422 drives the rotating member 2412 and the sliding member 2411 to be in a balanced state.

The limiting mechanism 24 is configured to abut against the non-limiting portion of the atomization mechanism 22. In some examples, after a limitation on the atomization mechanism 22 is released, a liquid pushing mechanism 26 may be used to push out the atomization mechanism 22 from the accommodating cavity, so that a position of the limiting portion of the atomization mechanism 22 changes with the atomization mechanism 22.

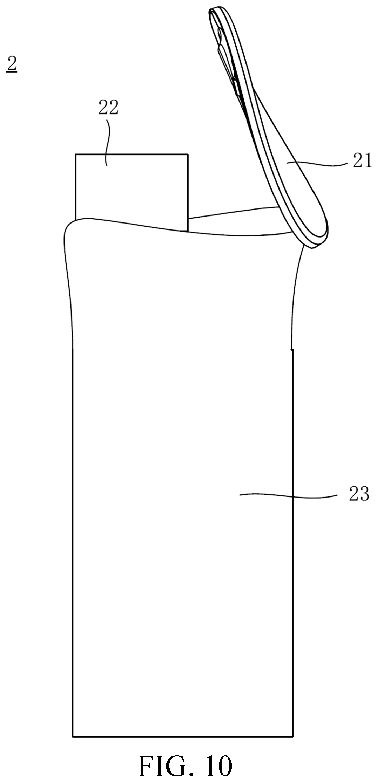

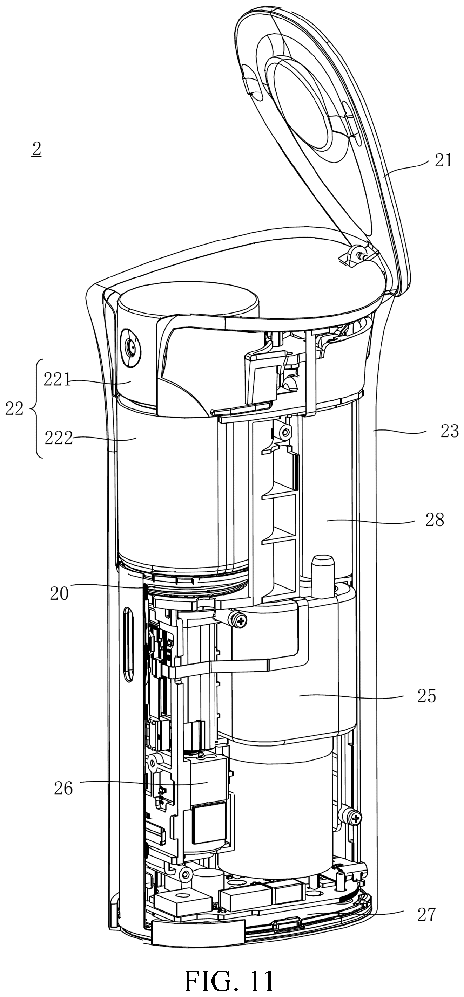

In some other examples, refer to FIG. 10 and FIG. 11. The atomization apparatus further includes a first elastic member 20. The first elastic member 20 is configured to be arranged in the accommodating cavity. For example, the first elastic member 20 is arranged in the accommodating cavity. The atomization mechanism 22 is mounted in the accommodating cavity. The atomization mechanism 20 presses the first elastic member 20, so that the first elastic member 20 generates a compressive elastic force. To be specific, the first elastic member 20 is configured to provide an elastic force for the atomization mechanism 22. During the opening coupling stroke, the limiting mechanism releases the limitation on the atomization mechanism 22, so that the compressive elastic force of the first elastic member 20 bounces the atomization mechanism 22 from the accommodating cavity, thereby changing the position of the limiting portion of the atomization mechanism 22, and further helping an operator take out the atomization mechanism 22. In addition, the limiting mechanism 24 abuts against the non-limiting portion of the atomization mechanism 22, thereby reducing the bouncing speed of the atomization mechanism 22, and avoiding a damage to the atomization mechanism 22 and the flip cover 21 because the atomization mechanism 22 collides with the flip cover 21. During the opening free stroke, the limiting mechanism 24 (for example, the limiting mechanism 24) may normally abut against the limiting portion of the atomization mechanism 22. Because the position of the limiting portion changes, the limiting mechanism 24 can only abut against another part (i.e., the non-limiting portion) of the atomization mechanism 22. To be specific, during the opening free stroke, the elastic force of the first elastic member 20 is used, so that the limiting mechanism 24 is configured to abut against the non-limiting portion of the atomization mechanism 22. The first elastic member 20 may be at least one of a leaf spring, a spring, and an elastic piece. An example in which the position of the limiting portion of the atomization mechanism 22 may be changed by using the first elastic member 20 is used for description below.

In some embodiments, in a covering process of the flip cover 21, the linkage fit between the flip cover 21 and the limiting mechanism 24 is non-connected coupling, so that the flip cover 21 switches a state of the limiting mechanism 24 from the release state to the limiting state. In this way, for example, during the opening free stroke, the position of the limiting portion of the atomization mechanism 22 changes. Therefore, when the flip cover 21 is closed, the limiting mechanism 24 may limit the atomization mechanism 22 in the accommodating cavity while the flip cover 21 may cover the mounting opening, thereby omitting the step in which the limiting mechanism 24 is separately operated for the atomization mechanism 22.

For example, in the closing process of the flip cover 21, the closing process of the flip cover 21 sequentially includes the closing coupling stroke and the closing empty stroke. For another example, in the closing process of the flip cover 21, the closing process of the flip cover 21 sequentially includes the closing free stroke, the closing coupling stroke, and the closing empty stroke. The closing free stroke, the closing coupling stroke, and the closing empty stroke of the flip cover 21 are described in detail below.

During the closing free stroke, the flip cover 21 is decoupled from the limiting mechanism 24, and the state of the limiting mechanism 24 is the release state.

The atomization mechanism 22 is mounted in the accommodating cavity. For example, the limiting mechanism 24 abuts against the non-limiting portion of the atomization mechanism 22, so that the state of the limiting mechanism 24 is the release state. Therefore, actions of the closing free stroke in the closing process and the opening free stroke in the opening process are opposite, and other actions are the same. To understand the closing free stroke, refer to a relevant description of the opening free stroke. The flip cover 21 (for example, the cover body 211) may be decoupled from the atomization mechanism 22, which is the closing free stroke, so that the atomization mechanism 22 can be conveniently mounted in the housing 23, to avoids interference from the flip cover 21. Certainly, the flip cover 21 may be in contact with the atomization mechanism 22.

During the closing coupling stroke, the flip cover 21 is coupled with the limiting mechanism 24, and the state of the limiting mechanism 24 is the release state. During the closing coupling stroke, the limiting mechanism 24 is in the release stroke, which may be understood as that the limiting mechanism 24 is separated from the atomization mechanism 22, and may also be understood as that the limiting mechanism 24 abuts against the non-limiting portion of the atomization mechanism 22.

In some examples, the rotating assembly 241 includes the rotating member 2412 and the sliding member 2411. The elastic assembly 242 includes the second elastic member 2422 and the third elastic member 2421. During the closing coupling stroke, the flip cover 21 is coupled with the rotating member 2412, the rotating member 2412 is decoupled from the sliding member 2411, and the third elastic member 2421 drives the sliding member 2411 to abut against the non-limiting portion of the atomization mechanism 22, so that the state of the limiting mechanism 24 is the release state. The second elastic member 2422 is configured to drive the rotating member 2412 to restore to the state in which the rotating member 2412 is just in contact with the sliding member 2411.

During the closing empty stroke, the flip cover 21 is coupled with the limiting mechanism 24. The flip cover 21 is configured to press the atomization mechanism 22, so that the state of the limiting mechanism 24 is switched to the limiting state.

Actions of the closing empty stroke in the closing process and the opening empty stroke in the opening process are opposite, and other actions are the same. Therefore, to understand the closing empty stroke, refer to a relevant description of the opening empty stroke. However, the limiting mechanism 24 abuts against the non-limiting portion of the atomization mechanism 22. During the closing empty stroke, the flip cover 21 is configured to press the atomization mechanism 22, so that the limiting portion of the atomization mechanism 22 restores (that is, the limiting portion is aligned with the limiting mechanism 24), and the limiting mechanism 24 is fitted with the limiting portion (for example, the latch is inserted into the groove 223), to switch the state of the limiting mechanism 24 to the limiting state.

In some embodiments, in the closing process of the flip cover 21, the linkage fit between the flip cover 21 and the limiting mechanism 24 is decoupling, and the state of the limiting mechanism 24 maintains the limiting state. In this way, the atomization mechanism 22 may be pressed manually or in another manner, so that the limiting portion of the atomization mechanism 22 is fitted with the limiting mechanism 24, to limit the atomization mechanism 22 in the accommodating cavity. Then, the flip cover 21 is closed to cover the mounting opening.

In this embodiment, the closing process may also include the closing free stroke, the closing coupling stroke, and the closing empty stroke. To understand the closing free stroke, the closing coupling stroke, and the closing empty stroke in this embodiment, refer to relevant descriptions of the closing free stroke, the closing coupling stroke, and the closing empty stroke in the foregoing embodiment. A difference exists between the closing empty stroke in this embodiment and the closing stroke in the foregoing embodiment. To be specific, during the closing empty stroke in this embodiment, the flip cover 21 does not press the atomization mechanism 22.

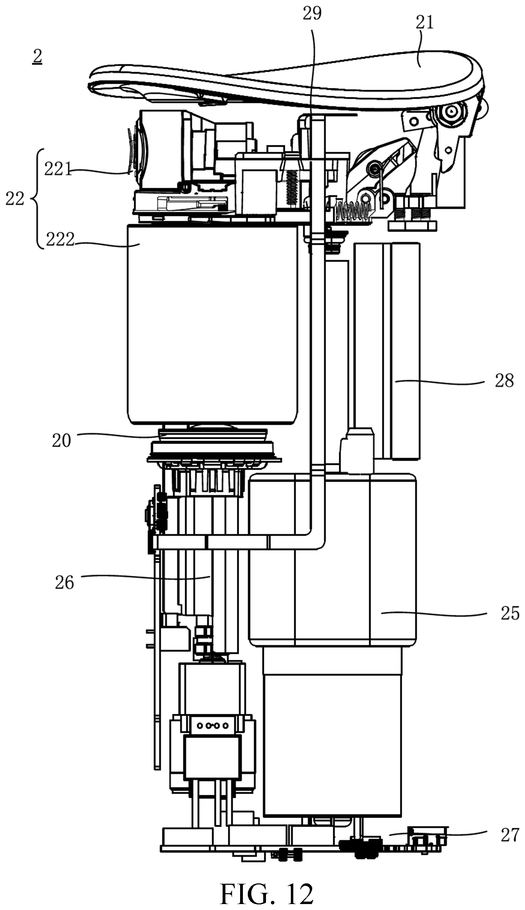

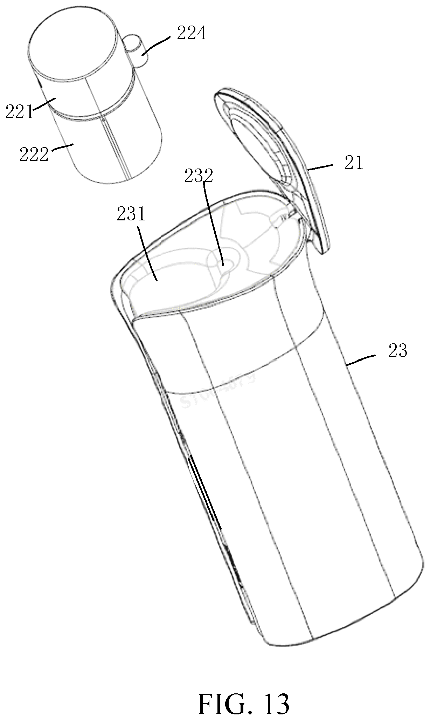

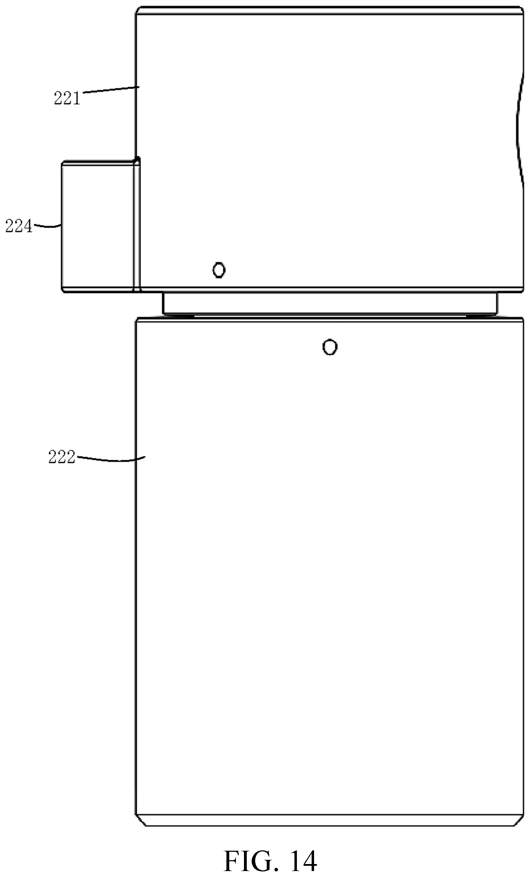

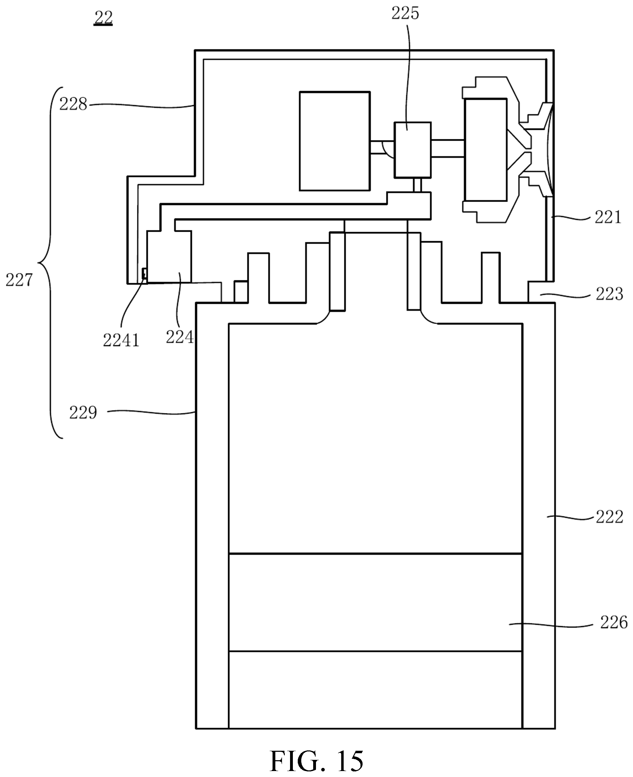

In some embodiments, refer to FIG. 11 to FIG. 15. FIG. 13 shows a structure of a part of the housing, so that a cooperation relationship between the housing and the air supply interface can be embodied. The atomization apparatus 2 further includes the air supply mechanism 25. The air supply mechanism 25 is configured to supply air to the atomization assembly 221. In this way, the air supplied by the air supply mechanism 25 may enter the atomization mechanism 22, to cause a liquid in the atomization mechanism 22 to be atomized and sprayed. The air supply mechanism 25 may include an air pump and a vent tube. The air pump is in communication with the atomization mechanism 22 (or the air supply interface 224) through the vent tube.

The air supply interface 224 protrudes from a first outer wall 227 of the atomization assembly 221, and the air supply mechanism 25 is in communication with the air supply interface 224. The air supply interface 224 protrudes from the first outer wall 227 of the atomization mechanism 22, which may provide a positioning and fool-proofing function.

The accommodating cavity 231 is provided with a positioning groove 232 fitted with the air supply interface 224. In this way, after the atomization mechanism 22 is mounted in the accommodating cavity 231, the air supply interface 224 is fitted with the positioning groove 232, to cause the atomization mechanism 22 to provide a positioning and fool-proofing function. In addition, a direction of a nozzle on the atomization mechanism 22 can also be positioned. In some examples, when the limiting mechanism 24 is fitted with the limiting portion of the atomization mechanism 22, the air supply interface 24 is also fitted with the positioning groove 232. For example, a top of the housing 23 has a mounting opening. The mounting opening may extend along a height direction of the housing from the top of the housing 23, to form the accommodating cavity 231 (that is, the accommodating cavity 231 may be referred to as an accommodating groove, and a notch of the accommodating groove may be the mounting opening). In this case, the top of the housing 23 further has the positioning groove 232 extending along the height direction of the housing from the top of the housing 23.

An inner wall of the air supply interface 224 has a slot 2241, and the air supply mechanism 25 has a protrusion; or the inner wall of the air supply interface 224 has a protrusion, and the air supply mechanism 25 has a slot. When the air supply mechanism 25 is in communication with the air supply interface 224, the protrusion is snap-fitted with the slot 2241. To be specific, the air supply mechanism 25 (for example, an air tube) is fixedly connected to and in communication with the air supply interface 224 by using a protrusion-slot structure.

For example, the atomization mechanism 22 may include the atomization assembly 221 and the atomization cartridge 222. A second outer wall 228 of the atomization assembly 221 and a third outer wall 229 of the atomization cartridge 222 form the first outer wall 227, and the air supply interface 224 protrudes from the second outer wall 228. In some examples, the second outer wall 228 is located in the third outer wall 229. To be specific, an orthographic projection of the third outer wall 229 on a first plane is located on an orthographic projection of the second outer wall 228 on the first plane, and the first plane is perpendicular to an installation direction (i.e., a direction of the elastic force of the first elastic member 20) of the atomization mechanism 22. In some other examples, the second outer wall 228 is flush with the third outer wall 229. Therefore, the air supply interface 224 provides a positioning and fool-proofing function.

The atomization cartridge 222 is in communication with the atomization assembly 221, so that the liquid in the atomization cartridge 222 can flow into the atomization assembly 221. In this way, a path between the atomization cartridge 222 and the atomization assembly 221 is relatively short, and less of the liquid is wasted.

The atomization cartridge 222 is detachably connected to the atomization assembly 221. For example, the atomization cartridge 222 is snap-fitted with or threadedly connected to the atomization assembly 221. The atomization assembly 221 has a liquid supply inlet, and the atomization cartridge 222 has a liquid supply outlet. The liquid supply inlet is in detachable communication with the liquid supply outlet, to implementation detachable connection between the atomization assembly 221 and the atomization cartridge 222. The atomization cartridge 222 or the atomization assembly 221 may be separately replaced by using the detachable connection, to reduce deterioration of the liquid in the atomization cartridge 222. In addition, different atomization assemblies 221 may also be matched for different liquids in the atomization cartridge 222, to maximize absorption of the liquids.

The atomization assembly 221 has an atomization cavity 225, which is configured to receive the liquid from the atomization cartridge 222 and the air from the air supply mechanism 25, so that the nozzle of the atomization assembly 221 can spray an atomized liquid. The atomization cartridge 222 is configured to store a liquid. The atomization cartridge 222 has a liquid storage cavity, which is configured to store a liquid.

The atomization apparatus 2 further includes the liquid pushing assembly. The liquid pushing assembly is configured to push the liquid in the atomization cartridge 222 into the atomization assembly 221.

The atomization assembly 221, the atomization cartridge 222, and the liquid pushing mechanism are sequentially arranged along an axial direction. In this way, the liquid pushing mechanism pushes the liquid in the atomization cartridge 222 into the atomization assembly 221 in a manner of a relatively simple structure. For example, an axial direction may be a height direction of the atomization apparatus. For another example, an axial direction may be a direction intersecting with the height direction of the atomization apparatus, and an angle between the two ranges from -10° to 10°.

The liquid pushing mechanism may include a push rod, a screw rod, a driving member, and a pressing plate. The driving member is connected to the screw rod, and the screw rod is threadedly connected to the push rod. The driving member is configured to drive the screw rod to rotate, to cause the push rod to move along an axis of the screw rod, so that the push rod pushes the liquid in the atomization cartridge 222 into the atomization assembly 221. In some examples, the driving member may include a motor and a speed change mechanism. The driving member is drive-connected with the speed change mechanism, and the speed change mechanism is connected to the screw rod. The speed change mechanism may be a speed reducer, a transmission, or the like.

The pressing plate is arranged on the driving member. For example, the pressing plate is fixed on the speed change mechanism. The pressing plate is rotatably connected to the screw rod. In this way, the push rod may first press the pressing plate, thereby avoiding a damage to the driving member caused by the push rod. In some examples, the pressing plate is fixed on the driving member and sleeved on an outer ring of a first bearing, and the screw rod is sleeved on an inner ring of the first bearing.

A piston 226 is arranged in the atomization cartridge 222. The liquid pushing mechanism 26 causes, through movement of the piston 226, the liquid in the liquid storage cavity of the atomization cartridge 222 to flow into the atomization cavity 225 of the atomization assembly 221.

In some examples, the piston 226 is directly connected to the liquid pushing mechanism 26. For example, the piston 226 is connected to the liquid pushing mechanism 26 through a connecting member (which may be a plate, a rod, or the like). In some other examples, the piston 226 is indirectly connected to the liquid pushing mechanism 26. For example, the piston 226 can abut against the liquid pushing mechanism 26.

The push rod is sleeved on an inner ring of a second bearing. For example, an outer ring of the second bearing abuts against the piston 226. For another example, the outer ring of the first bearing is connected to the piston 226 through the connecting member. Therefore, this avoids the push rod from rotating while driving the piston 226 to move.

In some embodiments, the atomization apparatus 2 further includes a first sensor 29 and a controller. The first sensor 29 is electrically connected to the controller. When the first sensor 29 detects that the atomization cartridge 222 is mounted in the accommodating cavity 231, the controller is configured to control the liquid pushing mechanism 26 to move toward the atomization cartridge 222, to reduce a time when the atomization apparatus waits for a liquid to flow out after being turned on. Certainly, when no atomization cartridge 222 is in the accommodating cavity 231, the liquid pushing mechanism 26 is in an initial state (which may be understood as that a movement amount of the liquid pushing mechanism 26 is zero or close to zero), or returns to the initial state.

In some embodiments, the atomization apparatus 2 further includes a second sensor and the controller. The first sensor 29 is electrically connected to the controller. When the second sensor detects that the liquid pushing mechanism 26 abuts against the piston 226, the controller is further configured to control the liquid pushing mechanism 26 to stop moving or return by a preset distance. An objective that a liquid quickly flows out is achieved after startup. For example, the piston 226 is provided with a magnet. When the sensor detects that the liquid pushing mechanism 26 is in contact with the magnet, the controller controls the liquid pushing mechanism 26 to stop moving or return by the preset distance.

In some examples, the first sensor 29 and the second sensor may be Hall sensors. The first sensor 29 and the second sensor are mounted in the housing 23. The first sensor 29 and the second sensor may be a same sensor, or may be two sensors. In this specification, an example in which the first sensor 29 and the second sensor are a same sensor (for example, the first sensor 29) is used for description.

The controller may be integrated on a circuit board 27. A switch may be mounted on the housing 23. The power supply 28 may be a secondary battery, for example, a lithium battery. The power supply 28 and the circuit board 27 may be mounted in the accommodating cavity 231 or the receiving cavity. In some examples, the circuit board 27 is mounted on an inner bottom wall of the housing 23, and the liquid pushing mechanism 26 and the air supply mechanism 25 are both located on the circuit board 27. The first elastic member 20 is located on the liquid pushing mechanism 26. The atomization cartridge 222 is located on the first elastic member 20. The atomization assembly 221 is located on the atomization cartridge 222. The power supply 28 is located on the air supply mechanism 25. The limiting mechanism 24 is located on the power supply 28. In this way, the components are compactly arranged, so that the housing 23 has a relatively small volume, thereby achieving an objective of a small footprint and convenience.

The foregoing descriptions are merely implementations of this application, and the patent protection scope of this application is not limited thereto. All equivalent structure or equivalent process changes made according to the content of this specification and the drawings in this application or by directly or indirectly applying this application in other related technical fields shall fall within the patent protection scope of this application.

## Claims

1. An atomization apparatus, comprising:
a housing, having an accommodating cavity and a mounting opening; and
an atomization mechanism, detachably mounted in the accommodating cavity.

2. The atomization apparatus of claim 1, further comprising an air supply mechanism, wherein the atomization apparatus is mounted in the accommodating cavity; an air supply interface protrudes from a first outer wall of the atomization mechanism; and the air supply mechanism is in communication with the air supply interface, and is configured to supply air to the atomization mechanism.

3. The atomization apparatus of claim 2, wherein the atomization mechanism comprises an atomization assembly and an atomization cartridge in communication with each other; liquid in the atomization cartridge is capable of entering the atomization assembly; and a second outer wall of the atomization assembly and a third outer wall of the atomization cartridge form the first outer wall, and the air supply interface protrudes from the second outer wall.

4. The atomization apparatus of claim 1, wherein the atomization mechanism comprises an atomization assembly and an atomization cartridge in communication with each other; the atomization assembly is detachably connected to the atomization cartridge.

5. The atomization apparatus of claim 1, wherein the atomization mechanism comprises an atomization assembly and an atomization cartridge in communication with each other; the atomization apparatus further comprises a liquid pushing mechanism mounted in the accommodating cavity; and the liquid pushing mechanism is configured to push the liquid in the atomization cartridge into the atomization assembly.

6. The atomization apparatus of claim 5, wherein the liquid pushing mechanism comprises a push rod, a screw rod, a driving member, and a pressing plate; the driving member is connected to the screw rod, and the screw rod is threadedly connected to the push rod; the driving member is configured to drive the screw rod to rotate, to cause the push rod to move along an axis of the screw rod; the push rod is configured to push the liquid in the atomization cartridge into the atomization assembly; and the pressing plate is arranged on the driving member, and is capable of abutting against the push rod.

7. The atomization apparatus of claim 5, wherein the atomization assembly, the atomization cartridge, and the liquid pushing mechanism are sequentially arranged along an axial direction.

8. The atomization apparatus of claim 5, wherein a piston is arranged in the atomization cartridge; and the liquid pushing mechanism is configured to push the piston to move.

9. The atomization apparatus of claim 1, wherein the atomization mechanism further comprises a flip cover mechanism, the flip cover mechanism covers a flip cover and a limiting mechanism, and the flip cover is rotatably connected to the housing and is configured to cover the mounting opening; the limiting mechanism has a limiting state and a release state; and a linkage fit between the flip cover and the limiting mechanism is non-connected coupling when the flip cover rotates, to cause the flip cover to switch a state of the limiting mechanism.

10. The atomization apparatus of claim 2, wherein the accommodating cavity is provided with a positioning groove mated with the air supply interface.
